# EUROPÄISCHE PATENTSCHRIFT

(11) **EP 3 287 131 B1**
(45) Veröffentlichungstag und Bekanntmachung des Hinweises auf die Patenterteilung: **04.03.2020**
(21) Anmeldenummer: 17189829.9
(22) Anmeldetag: 07.09.2012
(51) Int. Cl.: A61K 31/505, A61K 31/56, A61P 11/00

(54) **THERAPEUTISCHE ANWENDUNGEN VON ECTOIN**
THERAPEUTIC USES OF ECTOINE
APPLICATIONS THÉRAPEUTIQUES D'ECTOINE

(30) Priorität: 09.09.2011 DE 102011113059
(43) Veröffentlichungstag der Anmeldung: 28.02.2018
(62) Teilanmeldung aus: 12780649.5
(73) Patentinhaber: bitop AG, 58453 Witten (DE)
(72) Erfinder: Unfried, Klaus, 40225 Düsseldorf (DE); Sydlik, Ulrich, 41169 Mönchengladbach (DE); Krutmann, Jean, 40237 Düsseldorf (DE)
(74) Vertreter: Schöneborn, Holger

(56) Entgegenhaltungen:
- WO-A1-00/36915
- WO-A2-00/76528
- WO-A2-2009/027069
- DE-A1- 10 006 578
- BILSTEIN A ET AL: "Therapeutic effect of ectoine in an experimental model of allergic asthma", ALLERGY (OXFORD), Bd. 64, Nr. Suppl. 90, 2009, Seite 313, XP002689778, & 28TH CONGRESS OF THE EUROPEAN-ACADEMY-OF-ALLERGY-AND-CLINICAL-I MMUNOLOGY; WARSAW, POLAND; JUNE 06 -10, 2009 ISSN: 0105-4538
- SYDLIK U ET AL: "The compatible solute ectoine protects against nanoparticle-induced neutrophilic lung inflammation", TOXICOLOGY LETTERS,, Bd. 189, 13. September 2009 (2009-09-13), Seite S188, XP026420513, ELSEVIER BIOMEDICAL PRESS, AMSTERDAM, NL ISSN: 0378-4274 [gefunden am 2009-07-31]

## Beschreibung

Die Erfindung betrifft Zusammensetzungen, enthaltend Ectoin, Hydroxyectoin oder entsprechende Salze, Ester und Amide.

Osmolyte bzw. kompatible Solute aus extremophilen Mikroorganismen bilden eine bekannte Gruppe niedermolekularer Schutzstoffe. Extremophile sind sehr außergewöhnliche Mikroorganismen, da sie optimal bzw. bei hohen Salzkonzentrationen (bis 200 g NaCl/l) und hohen Temperaturen (60 bis 110°C) wachsen, die bei mesophilen (normalen) Organismen zu massiven Schädigungen zellulärer Strukturen führen würden. In den letzten Jahren wurde daher ein großer Forschungsaufwand betrieben, um die biochemischen Komponenten zu identifizieren, die zu der bemerkenswerten Stabilisierung der Zellstrukturen führen. Obwohl viele Enzyme aus hyperthermophilen Mikroorganismen auch unter hohen Temperaturen stabil sind, gilt dies nicht generell für die zellulären Strukturen thermo- und hyperthermophiler Organismen. Zu der hohen Temperaturstabilität von Zellstrukturen tragen in erheblichem Maße niedermolekulare organische Substanzen (kompatible Solute, Osmolyte) im intrazellulären Milieu bei. Verschiedene neuartige Osmolyte konnten in den letzten Jahren in extremophilen Mikroorganismen erstmals identifiziert werden. In einigen Fällen konnte der Beitrag dieser Verbindungen zum Schutz zellulärer Strukturen - vor allem Enzymen - gegenüber Hitze und Trockenheit bereits gezeigt werden (K. Lippert, E. A. Galinski, Appl. Microbiol. Biotech. 1992, 37, 61-65; P. Louis, H. G. Trüper, E. A. Galinski, Appl. Microbiol. Biotech. 1994, 41, 684-688; Ramos et al., Appl. Environm. Microbiol. 1997, 63, 4020-4025; Da Costa, Santos, Galinski, Adv. in Biochemical Engineering Biotechnology, 61, 117-153).

Für eine Reihe von kompatiblen Soluten haben sich sinnvolle Anwendungsmöglichkeiten im medizinischen, kosmetischen und biologischen Bereich ergeben. Zu den wichtigsten kompatiblen Soluten zählen dabei das Ectoin (2-Methyl-1,4,5,6-tetrahydropyrimidin-4-carbonsäure) und seine Derivate. So wird beispielsweise in der EP 0 887 418 A2 die Verwendung von Ectoin und Hydroxyectoin (5-Hydroxy-2-methyl-1,4,5,6-tetrahydropyrimin-4-carbonsäure) zur Behandlung von Hauterkrankungen oder als wirksamer Zusatz zur Kryoprotektion von biologischen Wirkstoffen und Zellen beschrieben. Die DE 10 2006 056 766 A1 zeigt die Verwendung von Ectoin zur Behandlung des Vascular Leak Syndroms (VLS). Weitere Beispiele sind die Stabilisierung von Vakzinen (DE 100 65 986 A1) oder die dermatologische Verwendung zur Behandlung von Neurodermitis (DE 103 30 243 A1).

Die Struktur des natürlichen L-Ectoins ((S)-2-Methyl-1,4,5,6-tetrahydropyrimidin-4-carbonsäure) ist im Folgenden dargestellt:

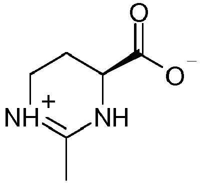

Auch das Hydroxyectoin wurde als für verschiedene Zwecke vorteilhaft beschrieben. Die Struktur des natürlichen Hydroxyectoins ((4S,5S)-5-Hydroxy-2-methyl-1,4,5,6-tetrahydropyrimidin-4-carbonsäure) wird im Folgenden wiedergegeben:

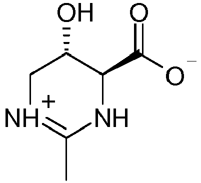

Die Behandlung von auf Schwebstaubeinwirkung beruhenden Lungenkrankheiten und kardiovaskulären Erkrankungen ist Gegenstand des europäischen Patents EP 1 641 442 B1. Hierin wird die Inhalation von Ectoin oder Hydroxyectoin enthaltenden Arzneimittelzubereitungen zur Bekämpfung derartiger Krankheiten beschrieben. Erkrankungen, die nicht auf Schwebstaubeinwirkung beruhen, sind jedoch nicht Gegenstand des Patents.

Bei vielen Entzündungserscheinungen spielen neutrophile Granulozyten, kurz Neutrophile, eine wichtige Rolle, insbesondere in der Bekämpfung von viralen und bakteriellen Pathogenen. Neutrophile werden in hoher Zahl im Knochenmark gebildet. Die Pathogene werden durch Freisetzung von reaktiven Sauerstoffspezies und Enzymen wie Myeloperoxidase, Elastase oder Matrixmetalloproteinasen zerstört. Da diese Reaktionen jedoch mit Nebenwirkungen auf das beteiligte Gewebe verbunden sind, muss eine strikte Regulation erfolgen, so dass die neutrophile Entzündung nicht über die Bekämpfung der eigentlichen Pathogene hinaus andauert. Entsprechend wird eine Signalkaskade aktiviert, die zur Apoptose der neutrophilen Granulozyten führt. Entzündungsmediatoren bewirken jedoch, dass die Apoptose hinausgezögert wird und verlängern auf diese Weise die Lebenszeit der Neutrophile. Die Akkumulation von Neutrophilen und Monozyten am Infektionsort stellt eine der Hauptkomponenten einer Entzündung dar. Die dauerhafte Verzögerung der Apoptose kann bis zu chronischen Entzündungserscheinungen führen. Beispiele sind eine chronische Lungenentzündung oder eine chronisch obstruktive Lungenerkrankung (COPD).

Der Schwerpunkt bei der Bekämpfung von chronischen Entzündungen liegt daher in der Bekämpfung der Entzündung, die auf die Akkumulation der Neutrophile zurückzuführen ist. Problematisch macht sich hierbei bemerkbar, dass Neutrophile, anders als andere an einer Entzündung beteiligte Zellen, auf Corticosteroide nur unbefriedigend ansprechen. Wünschenswert wären daher Medikamente, die die anti-apoptotische Wirkung von Entzündungsmediatoren, Corticosteroiden und anderen Stoffen einschränken.

Überraschend hat sich nunmehr herausgestellt, dass die Behandlung mit Ectoin oder Hydroxyectoin die anti-apoptotische Wirkung der Entzündungsmediatoren,

Corticosteroide und anderen Stoffen zumindest teilweise aufhebt und auf diese Weise die natürliche Apoptoserate von neutrophilen Granulozyten restauriert, ohne jedoch alleine eine pro-apoptotische Wirkung zu zeigen. Die Erfindung betrifft daher eine Zusammensetzung enthaltend Ectoin, Hydroxyectoin und/oder ein Salz, Ester oder Amid dieser Verbindungen zur Unterdrückung von anti-apoptotischen Signalen auf neutrophile Granulozyten und andere an Entzündungen beteiligte Zellen wie Makrophagen, eosinophile Granulozyten, basophile Granulozyten, Mastzellen, Lymphozyten, Epitheloidzellen und dendritische Zellen. Die Unterdrückung der anti-apoptotischen Signale steht normalerweise im Zusammenhang mit der Behandlung oder Prävention von Entzündungen, wobei chronische Entzündungen hier eine besondere Rolle spielen. Besondere Bedeutung hat die Bekämpfung von Entzündungen, die die Atemwege und die Lunge betreffen, insbesondere Lungenentzündungen, Asthma, chronisch obstruktive Lungenerkrankungen (COPD), ARDS, zystische Fibrose, Lungenfibrose, Silikose, Sarkoidose, Allergien und bronchiale Hyperreagibilität.

Die Hemmung der Apoptose der neutrophilen Granulozyten bei den untersuchten Entzündungsreaktionen wird auf eine membranvermittelte Aktivierung von membrangekoppelten Signalwegen über PI3-K (Phosphatidylinositol-3-kinase) zurückgeführt. Diese führen zu einer Aktivierung der Proteinkinase B (AKT) und letztlich zu einem Anstieg des Mcl-1-Levels, eines anti-apoptotisch wirkenden Proteins. Es wird vermutet, dass Ectoin die AKT-Aktivierung reduziert.

Die neutrophile Entzündungsreaktion wurde bei Ratten untersucht, denen intratracheal Kohlenstoffnanopartikel zugeführt wurden. Dies geschah sowohl zusammen mit als auch ohne Ectoin. Anschließend wurden die Ratten zu verschiedenen Zeitpunkten untersucht, wobei man nach zwei Tagen eine signifikante Reduzierung der Zahl an Neutrophilen in der Ectoin-Gruppe im Vergleich zur Placebo-Gruppe beobachten konnte. Die Wirksamkeit nach zwei Tagen ist in Übereinstimmung mit der beobachteten Reduzierung der cinc-1-Freisetzung, eines Chemokins, das bei Entzündungen eine wichtige Rolle spielt. Zunächst erfolgt die Freisetzung des cinc-1 in erster Linie durch Epithelzellen und Makrophagen, während zu späteren Zeitpunkten die Freisetzung durch die letztlich in hoher Zahl vorhandenen Neutrophile dominiert. Die Reduzierung der cinc-1-Freisetzung nach zwei Tagen bei Ectoin-Verabreichung zeigt, dass zu diesem Zeitpunkt die Zahl der Neutrophile abgenommen hat.

Ebenso konnte gezeigt werden, dass die Gabe von Ectoin in zwei Dosen 1 und 2 Tage nach Auslösung der Entzündungsreaktion praktisch denselben Effekt hat wie die Verabreichung des Ectoins bei Auslösung der Entzündungsreaktion. Ectoin kann somit nicht nur präventiv, sondern auch zur Behandlung einer bereits vorliegenden Entzündung eingesetzt werden. Auch bei wiederholter Gabe von Ectoin nach mehrfacher Auslösung einer Entzündungsreaktion konnte eine Reduzierung der Zahl an Neutrophilen sowie eine Absenkung des cinc-1-Niveaus beobachtet werden, was die Anwendbarkeit bei der Behandlung von chronischen Entzündungen unterstreicht.

Entsprechende Untersuchungen wurden auch mit isolierten humanen neutrophilen Granulozyten durchgeführt. Es konnte gezeigt werden, dass die Absenkung der Apoptoserate durch pro-entzündliche Faktoren wie Kohlenstoffnanopartikel (CNP), LTB₄ oder GM-CSF durch Gabe von Ectoin konzentrationsabhängig zumindest partiell kompensierbar ist. Die Gabe von Ectoin allein zu den Neutrophilen ohne vorherige Behandlung mit pro-entzündlichen Faktoren führte nicht zu einer Erhöhung der Apoptoserate. Dies zeigt, dass Ectoin nicht grundsätzlich pro-apoptotisch wirkt, sondern vielmehr die bei einer Entzündung ablaufenden anti-apoptotischen Mechanismen unterdrückt.

Die anti-anti-apoptotische Wirksamkeit wurde zwar anhand von Versuchen *in vivo* und *in vitro* nachgewiesen, bei denen eine Entzündungsreaktion mithilfe von Kohlenstoffnanopartikeln ausgelöst wurde, sie ist jedoch hierauf nicht beschränkt, vielmehr betrifft die vorliegende Erfindung explizit gerade auch solche Entzündungen, die nicht auf Schwebstaubeinwirkung zurückzuführen sind. Während die EP 1 641 442 B1 noch davon ausging, dass Ectoin nur direkt die schädlichen Auswirkungen von Schwebstäuben bekämpft, konnte nunmehr gezeigt werden, dass die Behandlung von Entzündungen mit Ectoin bei der Restauration der natürlichen Apoptoserate von Neutrophilen ansetzt.

Als besonders vorteilhaft hat sich darüber hinaus eine Kombination von Ectoin/Hydroxyectoin bzw. entsprechender Derivate mit Corticosteroiden erwiesen, insbesondere mit Glucocorticoiden wie Dexamethason, Budesonid, Betamethason, Triamcinolon, Fluocortolon, Methylprednisolon, Deflazacort, Prednisolon, Prednison, Cloprednol, Cortison, Hydrocortison, Fluocortin, Clocortolon, Clobetason, Alclomethason, Flumethason, Fluopredniden, Fluorandrenolon, Prednicarbat, Mometason, Methylprednisolon, Fluticason, Halomethason, Fluocinolon, Diflorasan, Desoximethason, Fluocinonid, Fludrocortison, Deflazacort, Rimexolon, Cloprednol, Amcinonid, Halcinonid, Diflucortolon, Clobetasol oder Salzen, Estern, Amiden, Solvaten oder Hydraten dieser Verbindungen.

Obgleich Corticosteroide als wirksame Mittel gegen Entzündungen bekannt sind, spielen sie bei der Bekämpfung von neutrophilen Entzündungen eine zweischneidige Rolle, da sie die natürliche neutrophile Apoptose herabsetzen. Durch Kombination eines Corticosteroids mit Ectoin/Hydroxyectoin wird daher die vorteilhafte antientzündliche Wirkung des Steroids mit der Restauration der natürlichen Apoptoserate und somit Reduktion der unerwünschten anti-apoptotischen Wirkung des Corticosteroids durch Ectoin/Hydroxyectoin kombiniert. Beispiele sind Kombinationen von Ectoin oder entsprechenden Derivaten mit Dexamethason und/oder Budesonid. Alternative vorteilhafte Kombinationen sind Ectoin/Hydroxyectoin mit GM-CSF, Leukotrienen wie LTB₄, Theophyllin (1,3-Dimethyl-xanthin), Leukotrienantagonisten, Phosphodiesterase-Hemmer (PDE-Hemmer, insbesondere PDE4-Hemmer), Muskarinrezeptor-Antagonisten, Anticholinergika wie Ipratropiumbromid oder Tiotropiumbromid oder anderen Arzneistoffen, bei denen die natürliche neutrophile Apoptoserate unerwünscht herabgesetzt wird.

Besondere Bedeutung hat auch bei einer Kombination von Corticosteroiden mit Ectoin/Hydroxyectoin die Behandlung von Lungenerkrankungen, insbesondere Lungenentzündungen, Asthma (Asthma bronchiale), chronisch obstruktiven Lungenerkrankungen (COPD), ARDS, zystischer Fibrose, Lungenfibrose, Silikose, Sarkoidose, Allergien und bronchialer Hyperreagibilität. Sinnvoll ist es dabei, die Zusammensetzung als inhalierbare Zusammensetzung vorzusehen. Die Zusammensetzung kann dabei flüssig als Lösung oder fest vorliegen, wobei die Zusammensetzung zweckmäßigerweise bei Bedarf mit Hilfe einer Inhalationsvorrichtung als Aerosol zerstäubt und eingeatmet wird.

Die Verabreichung von Corticosteroiden und Ectoin/Hydroxyectoin muss nicht in jedem Fall aus derselben Zusammensetzung heraus erfolgen, wichtig ist jedoch die gleichzeitige oder zeitnahe Verabreichung, so dass die Wirkstoffe funktionell in der oben beschriebenen Weise zusammenwirken. Entsprechend betrifft die Erfindung auch ein Kombinationspräparat, das zumindest zwei einzelne Zusammensetzungen umfasst, nämlich eine Zusammensetzung enthaltend Ectoin, Hydroxyectoin und/oder ein Salz, Ester oder Amid dieser Verbindungen sowie eine weitere Zusammensetzung, die ein Corticosteroid enthält. Das Kombinationspräparat stellt somit ein Kit of parts aus zwei Zusammensetzungen dar, die erst gemeinsam ihre volle Wirksamkeit entfalten. Bei dem Corticosteroid kann es sich insbesondere um eines der oben genannten Glucocorticoide handeln.

Als pharmakologisch verträgliche Salze des Ectoins/Hydroxyectoins kommen die Alkali- oder Erdalkalisalze, insbesondere die Salze des Kaliums, Natriums, Magnesiums und Calciums, aber auch Salze mit organischen Basen wie z. B. mit nicht toxischen aliphatischen oder aromatischen Aminen in Frage.

Durch Umsetzung der Carboxylgruppe des Ectoins/Hydroxyectoins mit Alkoholen oder Aminen, können entsprechende Ester oder Amide erhalten werden, die ebenfalls erfindungsgemäß einsetzbar sind. Im Falle eines Amids kann das Stickstoffatom wiederum gesättigte oder ungesättigte, geradkettige oder verzweigte Alkylgruppen aufweisen. Beim Hydroxyectoin kann auch die Hydroxygruppe mit einer Carbonsäure zu einem entsprechenden Ester umgesetzt sein.

Als vorteilhaft hat sich u. a. die Verwendung des Ectoinamids der 2-Hydroxy-5-aminobenzoesäure herausgestellt. Die Strukturformel ist im folgenden wiedergegeben:

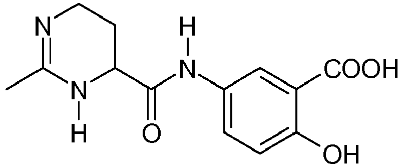

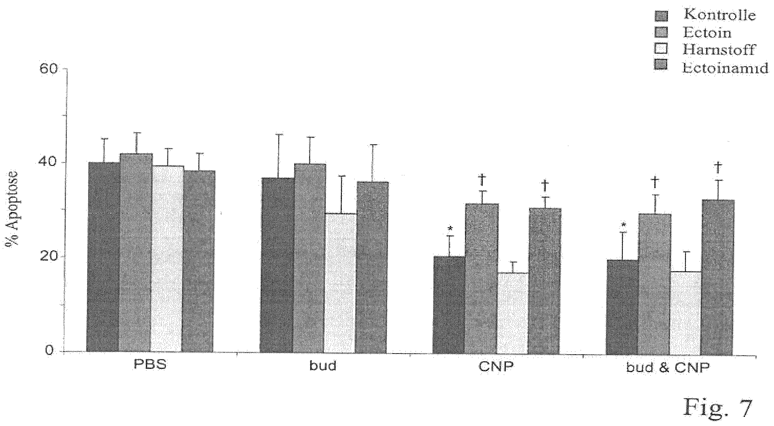

Es handelt sich somit um das 2-Methyl-1,4,5,6-tetrahydropyrimidin-4-carbonsäureamid der 2-Hydroxy-5-aminobenzoesäure. Bevorzugt handelt es sich um das entsprechende Amid des L-Ectoins: (S)-2-Methyl-1,4,5,6-tetrahydropyrimidin-4-carbonsäureamid. Die Verbindung wurde getestet und zeigte eine vergleichbare Wirksamkeit mit Ectoin selbst (vgl. Fig. 7). Möglich ist auch die Verwendung des entsprechenden Amids von Hydroxyectoin (5-Hydroxy-2-methyl-1,4,5,6-tetrahydropyrimin-4-carbonsäure), bevorzugt von L-Hydroxyectoin ((4S,5S)-5-Hydroxy-2-methyl-1,4,5,6-tetrahydropyrimidin-4-carbonsäure), d.h. das Hydroxyectoinamid der 2-Hydroxy-5-aminobenzoesäure. Auch das jeweilige Amid kann in ionischer oder zwitterionischer Form vorliegen. Die Erfindung betrifft somit auch die genannten Verbindungen bzw. Salze, Ester oder Amide dieser Verbindungen und Zusammensetzungen, die diese Verbindungen bzw. Salze, Ester oder Amide enthalten. Die Zusammensetzungen können der Verwendung als Arzneimittel dienen, insbesondere der Unterdrückung von anti-apoptotischen Signalen auf neutrophile Granulozyten, Makrophagen, eosinophile Granulozyten, basophile Granulozyten, Mastzellen, Lymphozyten, Epitheloidzellen, dendritische Zellen oder andere an Entzündungen beteiligte Zellen.

Allgemein können die erfindungsgemäßen Wirkstoffe ggf. auch mit weiteren Wirkstoffen unter Einsatz von pharmakologisch unbedenklichen Hilfs- und Zusatzstoffen zu vorzugsweise inhalierbaren Arzneimitteln verarbeitet werden. Solche Zusätze sind bei inhalierbaren flüssigen Zubereitungen in erster Linie Wasser, ggf. unter Zusatz von weiteren Lösungsmitteln, Stabilisatoren, Konservierungsmitteln, Emulgatoren, Antioxidantien, Füllstoffen oder Lösungsvermittlern. Als weitere Wirkstoffe sind Antiasthmatika, Broncholytika, nichtsteroidale antientzündliche Stoffe (NSAIDs) oder Expektorantia denkbar. Als Konservierungsmittel kommen in Frage: Benzalkoniumchlorid, Chlorbutanol, Thiomersal, Methylparaben, Propylparaben, Sorbinsäure und deren Salze, Natriumedetat, Phenylethylalkohol, Chlohexidinhydrochloridacetat, -digluconat, Cetylpyridiniumchlorid, -bromid, Chlorkresol, Phenylquecksilberacetat, Phenylquecksilbernitrat, Phenylquecksilberborat, Phenoxyethanol.

Die Formulierungen der Erfindung können ebenfalls geeignete Puffersysteme oder andere Hilfsstoffe zur pH-Einstellung beeinhalten, um einen pH-Wert einzustellen und aufrechtzuerhalten in der Größenordnung von 4-8, vorzugsweise von 5 bis 7,5. Geeignete Puffersysteme sind Citrat, Phosphat, Trometamol, Glycin, Borat, Acetat. Diese Puffersysteme können hergestellt werden aus Substanzen wie Citronensäure, Mononatriumphosphat, Dinatriumphosphat, Glycin, Borsäure, Natriumtetraborat, Essigsäure oder Natriumactat.

Die Konzentration des Ectoins/Hydroxyectoins bzw. entsprechenden Derivats bezogen auf die Zusammensetzung beträgt typischerweise 0,001 bis 50 Gew.-%, bevorzugt 0,05 bis 20 Gew.-%, insbesondere 0,1 bis 10 Gew.-%.

Im Fall der Verabreichung in fester Form, bspw. mittels Pulverinhalatoren, ist es sinnvoll, nur leicht resorbierbare nichtreizende Trägerstoffe wie mikronisierte Lactose einzusetzen.

### Versuch 1

Kohlenstoffnanopartikel (CNP, 14 nm, Printex 90, Degussa, Frankfurt, Deutschland) wurden mit Hilfe einer Ultraschallbehandlung in einer phosphatgepufferten Salzlösung (PBS) suspendiert. Ebenso wurde eine 0,1 und eine 1 mM-Lösung von Ectoin in PBS hergestellt. Weibliche Fisher 344 Ratten wurden intratracheal mit 0,4 ml der CNP-Partikelsuspension behandelt. Nach 1 und 2 Tagen fand eine Behandlung mit 0,4 ml der Ectoin-Lösungen bzw. PBS statt. Am 3. Tag wurden die Ratten getötet, die Lungen wurden mit je 4 x 5 ml PBS gespült. Die Zellen jedes Tieres wurden in 1 ml PBS suspendiert und zentrifugiert. Die Pellets wurden 1 mal mit PBS gewaschen und in 300 µl hypotonischer Lösung (0,1 % Natriumcitrat, 0,1 % Triton X 100) enthaltend 50 µg/ml Propidiumiodid (PI) resuspendiert. Anschließend erfolgte die Fluoreszenzmessung zur Bestimmung der Apoptoserate.

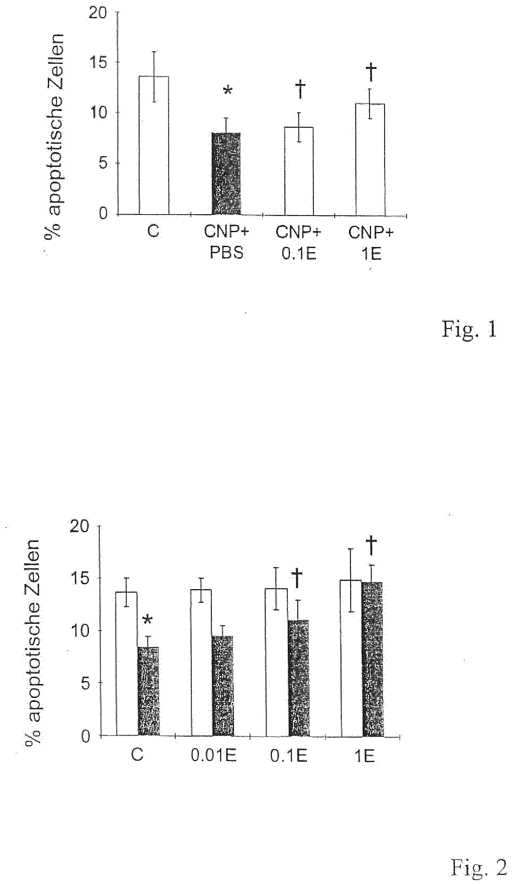

Man erhielt das in Figur 1 dargestellte Ergebnis (C: Kontrolle ohne CNP-Behandlung; *: signifikanter Unterschied zur Kontrollgruppe ohne CNP-Behandlung; †: signifikanter Unterschied zu nur mit CNP und PBS behandelten Tieren).

### Versuch 2

Die Wirkung von Ectoin auf die Apoptose wurde anhand von menschlichen Neutrophilen untersucht. Neutrophile von 3 männlichen und 2 weiblichen jungen, gesunden Spendern wurden isoliert und mit den angegebenen Mengen Ectoin (mM) behandelt. Die Behandlung erfolgte mit Ectoin allein (offene Balken) bzw. mit 33 µg/ml CNP (schwarze Balken). In der Kontrollgruppe (C) wurde kein Ectoin verabreicht. Das Ergebnis ist in Figur 2 dargestellt.

Die Quantifizierung der apoptotischen Zellen erfolgte in folgender Weise: Die Neutrophile wurden in 300 µl hypotonischer Lösung enthaltend Propidiumiodid (PI) suspendiert. Die Fluoreszenz von PI wurde über Durchflusszytometrie gemessen (FACScan Zytometer, BD Biosciences). Die Ergebnisse werden dargestellt als Prozentsatz an hypodiploider DNA (sub-G1), korrespondierend zur fragmentierten DNA, die für apoptotische Zellen charakteristisch ist.

Die durch CNP hervorgerufene Reduktion der Apoptoserate konnte durch Verabreichung signifikanter Mengen Ectoin praktisch aufgehoben werden.

(*: signifikanter Unterschied zur Kontrollgruppe ohne CNP-Behandlung; †: signifikanter Unterschied zu nur mit CNP und PBS behandelten Neutrophilen).

### Versuch 3

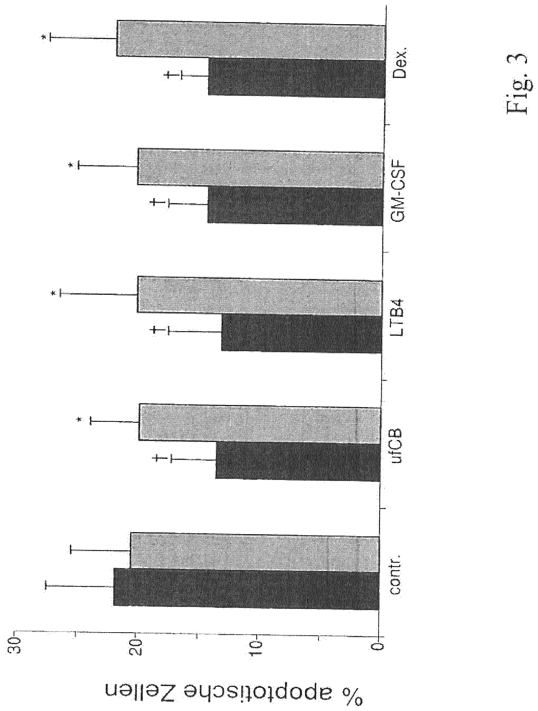

Die Wirkung von Ectoin auf die Apoptose wurde anhand von menschlichen Neutrophilen untersucht. Neutrophile von 3 männlichen und 2 weiblichen jungen, gesunden Spendern wurden isoliert und für 2 h mit PBS (dunkle Balken) bzw. mit 1 mM Ectoin (helle Balken) vorbehandelt. Anschließend erfolgte die Behandlung mit 33 µg/ml Kohlenstoffnanopartikeln (ufCB: ultrafine carbon black), 300 nM LTB₄, 20 ng/ml GM-CSF oder 1 µM Dexamethason bzw. keine Behandlung mit pro-entzündlichen Faktoren. Das Ergebnis ist in Figur 3 dargestellt. (†: signifikanter Unterschied zur Kontrollgruppe; *: signifikanter Unterschied zu nur mit pro-entzündlichen Faktoren und PBS behandelten Neutrophilen, Quantifizierung der apoptotischen Zellen analog Versuch 2).

### Versuch 4

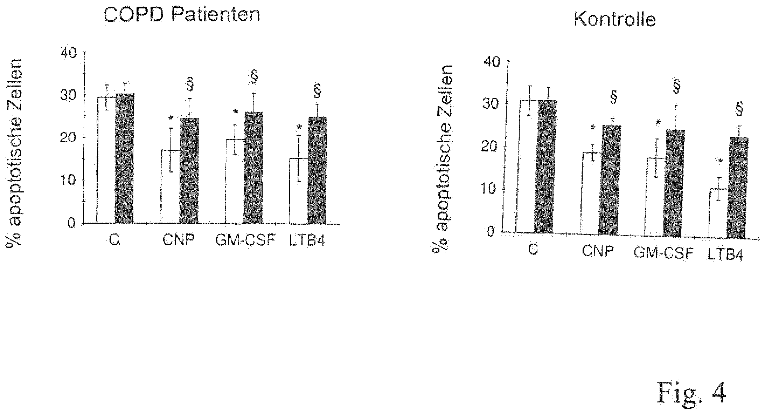

Die Wirkung von Ectoin auf die Apoptose wurde analog zu Versuch 3 bei COPD-Patienten und entsprechend alten Nicht-COPD-Patienten nachgewiesen. Die neutrophilen Granulozyten wurden für 2 h mit 1 mM Ectoin bzw. PBS vorbehandelt, anschließend folgte Behandlung für 16 h mit 33 µg/ml CNP, 300 nM LTB₄, 20 ng/ml GM-CSF oder PBS. Es konnte eine höhere Basis-Apoptose festgestellt werden, dennoch reduzierten die inflammatorischen Stimulantien die Apoptose und eine zusätzliche Behandlung mit Ectoin restaurierte die Apoptoserate signifikant. Das Ergebnis ist in Figur 4 dargestellt. (dunkle Balken: Vorbehandlung mit Ectoin; helle Balken: Vorbehandlung mit PBS; *: signifikanter Unterschied zur Kontrollgruppe ohne CNP oder Entzündungsmediatoren; §: signifikanter Unterschied zur Behandlung ohne Ectoin; Quantifizierung der apoptotischen Zellen analog Versuch 2).

### Versuche 5-7

Für die Versuche 5 bis 7 wurden neutrophile Granulozyten aus Blutproben gewonnen. Die Gruppen 1 und 2 bestanden aus Personen aus einer laufenden Patientenstudie. Männliche Patienten (Alter: 40 bis 80 Jahre) mit stabilem COPD-Verlauf (GOLD III/IV) und gesunden Kontrollpatienten in der gleichen Altersgruppe.

Darüber hinaus wurden junge, männliche Freiwillige in der Klinik (Gruppe 3) herangezogen.

### Versuch 5

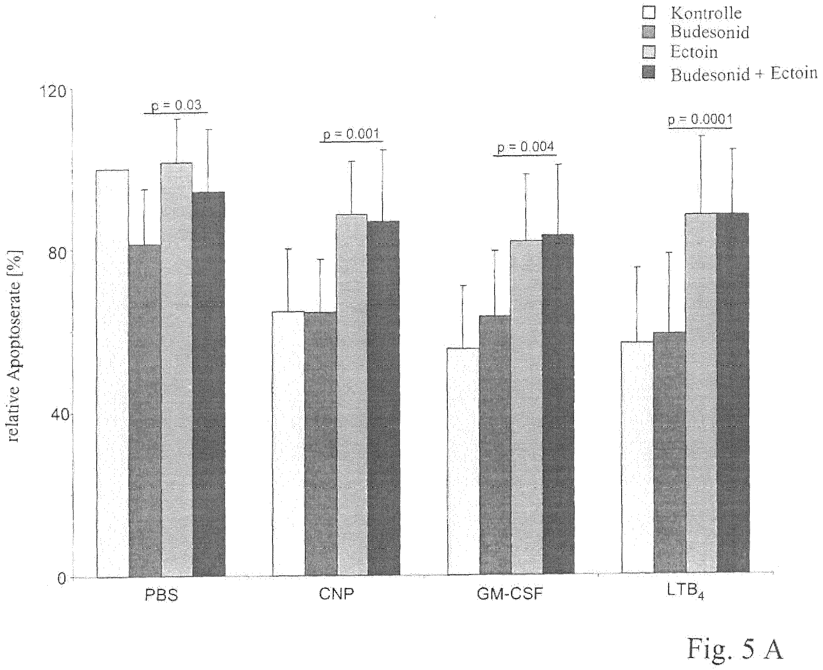

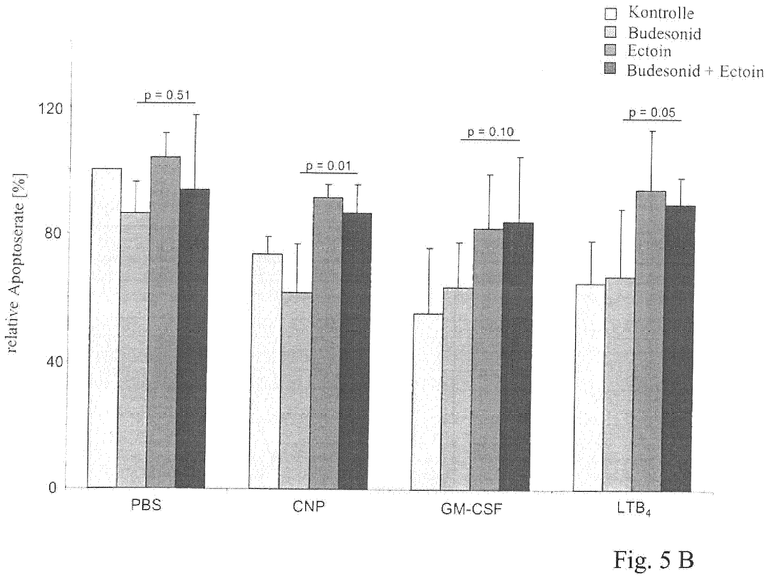

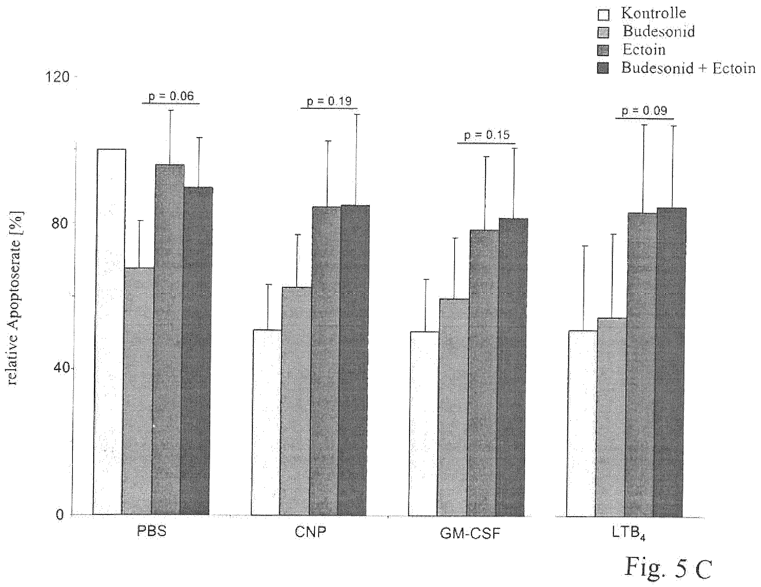

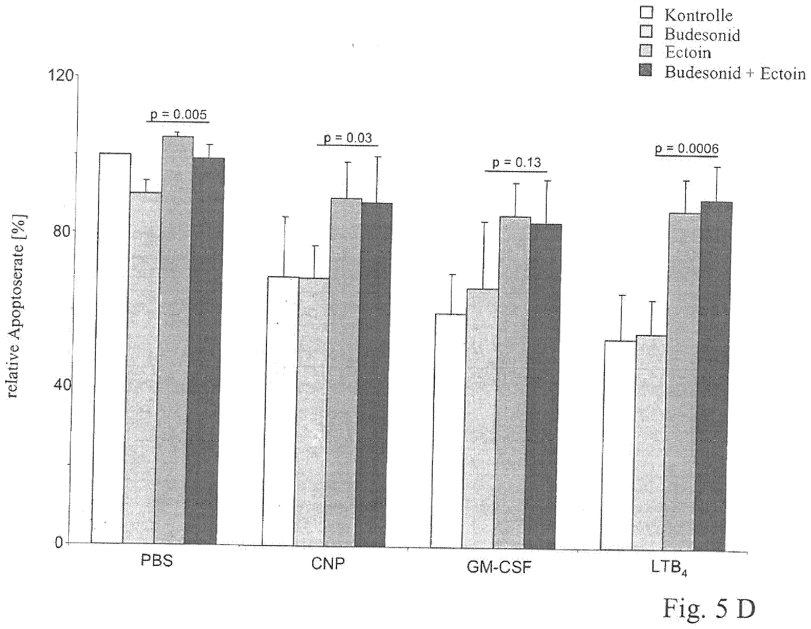

Der Einfluss von Ectoin in Kombination mit dem Corticosteroid Budesonid auf Apoptoseraten von Neutrophilen wird in Figur 5 dargestellt. Es wurden sub-G₁-Zellen nach Propidiumiodid-Färbung (FACS-Durchflusszytometrie) gemessen. Zellen: primäre, periphere Neutrophile. Isolierung der Neutrophile durch Percoll®-Zentrifugation. Kultivierung von 2 x 10⁶ Neutrophilen in Gegenwart von 33 µg/ml CNP, 300 nM LTB₄, 20 ng/ml GM-CSF, 1 µM Budesonid, 1 mM Ectoin und Kombinationen hieraus für 16 h. Figur 5 A: Zellen aus allen Proben kumuliert (n = 15), Figur 5 B: Zellen aus COPD-Patienten (n = 5), Figur 5 C: Zellen aus gesunder Alterskontrolle (n = 5), Figur 5 D: Zellen aus jungen Freiwilligen (n = 5).

Die Behandlung mit Budesonid führt zu einer Abnahme der Apoptoserate von Neutrophilen. Man erkennt, dass die Vorbehandlung der Zellen mit 1 mM Ectoin den anti-apoptotischen Effekten des Budesonids signifikant vorbeugt. Der Effekt trat in sämtlichen Gruppen als auch in der Gesamtheit der Gruppen auf. Der Effekt konnte bei neutrophilen Granulozyten gezeigt werden, die nicht mit pro-entzündlich wirkenden Stoffen behandelt wurden. Aber auch bei der Kombination von pro-entzündlich wirkenden Stoffen (CNP, LTB₄, GM-CSF) mit Budesonid verhinderte Ectoin die anti-apoptotische Wirkung erfolgreich.

### Versuch 6

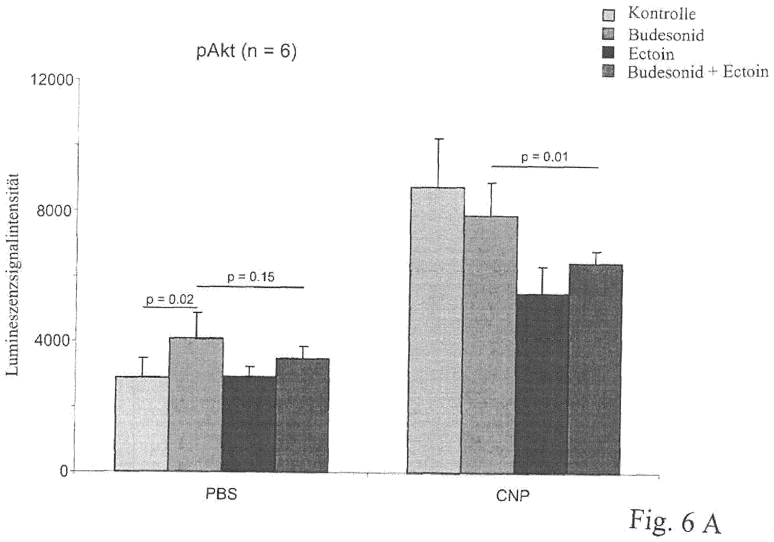

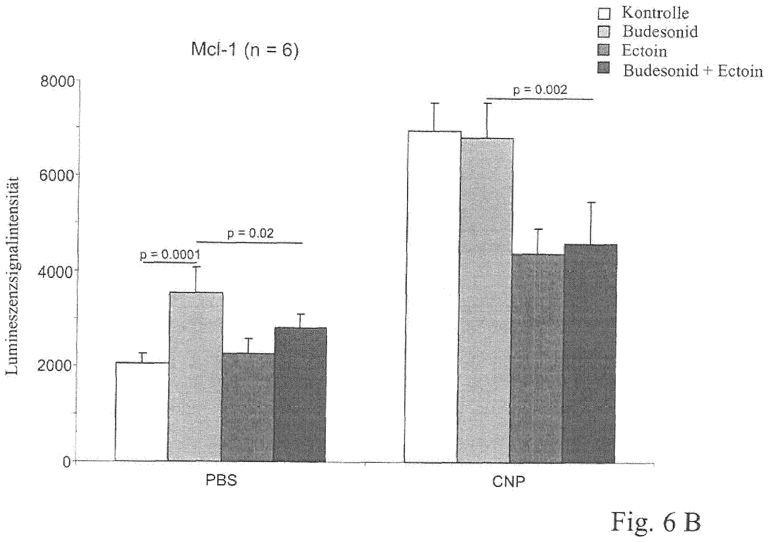

Der Einfluss von Ectoin in Kombination mit Budenosid auf anti-apoptotische Signale ist Figur 6 zu entnehmen. Die entsprechenden Signale über die Proteinkinase B (Akt) und Mcl-1 wurden bei ausgewählten Proben von Neutrophilen über Messung der Akt-Phosphorylierung und des Mcl-1-Proteinlevels durchgeführt, als Zellen dienten humane primäre, periphere Neutrophile. Isolierung der Neutrophile durch Percoll®-Zentrifugation, die Kultivierung von 2 x 10⁶ Neutrophilen in Gegenwart von 33 µg/ml CNP, 1 µM Budesonid, 1 mM Ectoin und Kombinationen hieraus für 6 h. Es erfolgte eine Proteinisolierung, ein Western Blot, Lumineszenz auf Röntgenfilmen an Material von jeweils 3 COPD-Patienten und 3 Personen aus der entsprechenden Alterskontrolle. Das Ergebnis zeigt deutlich den anti-apoptotischen Effekt von Budesonid, das den Akt-Signalweg aktiviert und die Menge an antiapoptotischem Protein Mcl-1 erhöht. Ectoin ist in der Lage, diesem Effekt entgegenzuwirken, auch in der Gegenwart von CNP.

### Versuch 7

Der Einfluss verschiedener weiterer Testsubstanzen auf die Apoptoserate von menschlichen Neutrophilen ist in Figur 7 dargestellt. Es wurden sub-G₁-Zellen nach Propidiumiodid-Färbung (FACS-Durchflusszytometrie) gemessen. Zellen: primäre, periphere humane Neutrophile. Isolierung der Neutrophile durch Percoll®-Zentrifugation und Kultivierung von 2 x 10⁶ Neutrophilen in Gegenwart von 33 µg/ml CNP, 1 µM Budesonid, 1 mM Ectoin, 1 mM Harnstoff, 1 mM Ectoinamid und Kombinationen hieraus für 16 h. Die Zellen stammen von jungen Freiwilligen (Gruppe 3), n = 5, bud = Budenosid. Zwei weitere Testsubstanzen (Harnstoff und das Ectoinamid von 2-Hydroxy-5-aminobenzoesäure) wurden auf ihre Fähigkeiten hin getestet, anti-apoptotische Effekte auf Neutrophile zu unterbinden. Beide Substanzen hatten keinen Einfluss auf die Hintergrundapoptoserate. Ectoinamid war in der Lage, die Reduktion der Apoptoserate, die durch Kohlenstoffnanopartikel (CNP) mit oder ohne Budenosid hervorgerufen wurde, zu verhindern, Harnstoff hingegen nicht.

## Patentansprüche

1. Zusammensetzung enthaltend Ectoin, Hydroxyectoin und/oder ein Salz, Ester oder Amid dieser Verbindungen zur Verwendung in einem Verfahren zur Behandlung oder Prävention von Lungenerkrankungen, ausgenommen solchen, die auf die Einwirkung von Schwebstäuben zurückzuführen sind, und ausgenommen Allergien und Asthma, wobei die Lungenerkrankung bronchiale Hyperreagibilität ist.

2. Zusammensetzung zur Verwendung nach Anspruch 1, **dadurch gekennzeichnet, dass** die Zusammensetzung zumindest ein Corticosteroid enthält.

3. Zusammensetzung zur Verwendung nach Anspruch 2, **dadurch gekennzeichnet, dass** das Corticosteroid ein Glucocorticoid ist.

4. Zusammensetzung zur Verwendung nach Anspruch 3, **dadurch gekennzeichnet, dass** das Glucocorticoid Dexamethason, Budesonid, Betamethason, Triamcinolon, Fluocortolon, Methylprednisolon, Deflazacort, Prednisolon, Prednison, Cloprednol, Cortison, Hydrocortison, Fluocortin, Clocortolon, Clobetason, Alclomethason, Flumethason, Fluopredniden, Fluorandrenolon, Prednicarbat, Mometason, Methylprednisolon, Fluticason, Halomethason, Fluocinolon, Diflorasan, Desoximethason, Fluocinonid, Fludrocortison, Deflazacort, Rimexolon, Cloprednol, Amcinonid, Halcinonid, Diflucortolon, Clobetasol oder ein Salz, Ester, Amid, Solvat oder Hydrat einer der vorgenannten Verbindungen ist.

5. Zusammensetzung zur Verwendung nach einem der Ansprüche 1 bis 4, **dadurch gekennzeichnet, dass** die Zusammensetzung eine inhalierbare Zusammensetzung ist.

6. Kombinationspräparat aus Zusammensetzungen zur Anwendung in einem Verfahren zur Prävention und/oder Behandlung von Lungenerkrankungen, ausgenommen Allergien und Asthma, wobei die Lungenerkrankung bronchiale Hyperreagibilität ist und die Zusammensetzungen zur zeitnahen Verabreichung vorgesehen sind, bestehend aus zumindest
- einer ersten Zusammensetzung enthaltend Ectoin, Hydroxyectoin und/oder ein Salz, Ester oder Amid dieser Verbindungen und
- einer zweiten Zusammensetzung enthaltend ein Corticosteroid.

## Claims

1. Composition comprising ectoine, hydroxyectoine and/or a salt, ester or amide of these compounds for use in a method of treatment or prevention of pulmonary diseases except those attributable to the influence of airborne particulate matter, and except allergies and asthma, wherein the pulmonary disease is bronchial hyperresponsiveness.

2. Composition for use according to claim 1, **characterized in that** the composition comprises at least one corticosteroid.

3. Composition for use according to claim 2, **characterized in that** the corticosteroid is a glucocorticoid.

4. Composition for use according to claim 3, **characterized in that** the glucocorticoid is dexamethasone, budesonide, betamethasone, triamcinolone, fluocortolone, methylprednisolone, deflazacort, prednisolone, prednisone, cloprednole, cortisone, hydrocortisone, fluocortine, clocortolone, clobetasone, alclomethasone, flumethasone, fluoprednidene, fluorandrenolone, prednicarbate, mometasone, methylprednisolone, fluticasone, halometasone, fluocinolone, diflorasone, desoximetasone, fluocinonide, fludrocortisone, deflazacort, rimexolone, cloprednole, amcinonide, halcinonide, diflucortolone, clobetasol or a salt, ester, amide, solvate or hydrate of one of the above mentioned compounds.

5. Composition for use according to any of claims 1 to 4, **characterized in that** the composition is an inhalable composition.

6. Combination preparation of compositions for use in a method of prevention and/or treatment of pulmonary diseases, except allergies and asthma, wherein the pulmonary disease is bronchial hyperresponsiveness and the compositions are provided for administration within a narrow time frame, consisting of at least
- a first composition comprising ectoine, hydroxyectoine and/or a salt, ester or amide of these compounds and
- a second composition comprising a corticosteroid.

## Revendications

1. Composition contenant de l'ectoïne, de l'hydroxyectoïne et/ou un sel, de l'ester ou de l'amide desdits composés destinée à être utilisée dans un procédé de traitement ou de prévention de maladies pulmonaires, à l'exception de celles, qui sont liées à l'effet de poussières en suspension, et à l'exception d'allergies et de l'asthme, dans laquelle la maladie pulmonaire est une hyperréactivité bronchique.

2. Composition destinée à être utilisée selon la revendication 1, **caractérisée en ce que** la composition contient au moins un corticostéroïde.

3. Composition destinée à être utilisée selon la revendication 2, **caractérisée en ce que** le corticostéroïde est un glucocorticoïde.

4. Composition destinée à être utilisée selon la revendication 3, **caractérisée en ce que** le glucocorticoïde est de la dexaméthasone, du budésonide, de la bétaméthasone, du triamcinolone, de la fluocortolone, de la méthylprednisolone, du deflazacor, de la prednisolone, de la prednisone, du cloprednol, de la cortisone, de l'hydrocortisone, de la fluocortine, de la clocortolone, de la clobétasone, de l'alclométhasone, de la fluméthasone, du fluprednidène, du fluoroandrenolone, du prednicarbate, de la mométasone, de la méthylprednisolone, de la fluticasone, de l'halométhasone, de la fluocinolone, de la diflorasone, de la désoximéthasone, du fluocinonide, de la fludrocortisone, du deflazacort, de la riméxolone, du cloprednol, de l'amcinonide, de l'halcinonide, du diflucortolone, du clobetasol ou un sel, de l'ester, de l'amide, un solvate ou de l'hydrate d'un des composés susmentionnés.

5. Composition destinée à être utilisée selon l'une quelconque des revendications 1 à 4, **caractérisée en ce que** la composition est une composition pouvant être inhalée.

6. Préparation combinée composée de compositions destinée à être mise en œuvre dans un procédé de prévention et/ou de traitement de maladies pulmonaires, à l'exception d'allergies et de l'asthme, dans laquelle la maladie pulmonaire est une hyperréactivité bronchique et les compositions sont prévues pour l'administration rapprochée, constituée d'au moins
- une première composition contenant de l'ectoïne, de l'hydoxyectoïne et/ou un sel, de l'ester ou de l'amide desdits composés, et
- une deuxième composition contenant un corticostéroïde.
